# EUROPÄISCHE PATENTSCHRIFT

(11) **EP 1 015 621 B1**
(45) Veröffentlichungstag und Bekanntmachung des Hinweises auf die Patenterteilung: **09.03.2005**
(21) Anmeldenummer: 98954301.2
(22) Anmeldetag: 30.09.1998
(51) Int. Cl.: C12P 13/20, C12N 15/60

(54) **VERFAHREN ZUR MIKROBIELLEN HERSTELLUNG VON AMINOSÄUREN DER ASPARTAT- UND/ODER GLUTAMATFAMILIE UND IM VERFAHREN EINSETZBARE MITTEL**
METHOD FOR MICROBIAL PRODUCTION OF AMINO ACIDS OF THE ASPARTATE AND/OR GLUTAMATE FAMILY AND AGENTS WHICH CAN BE USED IN SAID METHOD
PROCEDE DE PREPARATION MICROBIENNE D'AMINOACIDES DE LA FAMILLE DE L'ASPARTATE ET/OU DU GLUTAMATE ET AGENTS UTILISABLES DANS LE CADRE DE L'APPLICATION DUDIT PROCEDE

(30) Priorität: 04.10.1997 DE 19743894; 14.07.1998 DE 19831609
(43) Veröffentlichungstag der Anmeldung: 05.07.2000
(73) Patentinhaber: Degussa AG, 40474 Düsseldorf (DE)
(72) Erfinder: EIKMANNS, Bernd, D-89081 Ulm (DE); PETERS-WENDISCH, Petra, D-51496 Bergisch-Gladbach (DE); SAHM, Hermann, D-52428 Jülich (DE)
(86) Internationale Anmeldenummer: PCT/EP1998/006210
(87) Internationale Veröffentlichungsnummer: WO 1999/018228

(56) Entgegenhaltungen:
- EP-A1- 0 723 011
- Microbiology, Band 144, 1998, Petra G. Peters-Wendisch et al, "Pyruvate carboxylase from Corynebacterium glutamicum: characterization, expression and inactivation of the pyc gene", XP002110208
- EMBL, Databas Genbank/DDBJ, accession no. Y09548, Peters-Wendisch P.G. et al: "Pyruvate carboxylase from Corynebacterium glutamicum: characterization, expression and inactivation of the pyc gene", & 11 Feb 1998, nt 20-109, 165-3587, XP002900635
- Appl Microbiol Biotechnol, Band 47, 1997, S.M. Park et al, "Elucidation of anaplerotic pathways in Corynebacterium glutamicum via 13C-NMR spectroscopy and GC-MS", XP002900636
- Microbiology, Band 143, 1997, Petra G. Peters-Wendisch et al, "Pyruvate carboxylase as an anaplerotic enzyme in Corynebacerium glutamicum", XP002110209
- Chemical Abstracts, Band 126, Nr 12, 24 März 1997, (Columbus, Ohio, US), Peters-Wendisch, Petra, "Anaplerotic reactions in Corynebacterium glutamicum. Studies of the significance of phosphoenolpyruvate (PEP)-carboxylase and pyruvate-carboxylase in the central metabolism and in amino acid production",, Zusammenfassung 154946, Ber. Forschungszent. Juelich 1996, 1-121, XP002900637
- Applied and Environmental Microbiology, Band 62, Nr. 2, Februar 1996, Muriel Cocaign-Bousquet et al, "Growth Rate-Dependent Modulation of Carbon Flux through Central Metabolism and the Kinetic Consequences for Glucose-Limited Chemostat Cultures of Corynebacterium glutamicum", XP002900638
- Databas Pir, accession no. S73055, R. Smith et al: "Mycobacterium tuberculosis cosmid tbc2", EMBL Data Library, September 1994, a.a. 1-1144, XP002900639

## Beschreibung

Die Erfindung betritt ein Verfahren zur mikrobiellen Herstellung von Aminosäuren der Aspartat- und/oder Glutamatfamilie gemäß den Ansprüchen 1 bis 17, Pyruvat-Carboxylase-Gene nach Anspruch 18 bis 23, Genstrukturen nach Anspruch 24, Vektoren nach Anspruch 25, transformierte Zellen nach Anspruch 26 bis 31 sowie Verwendungen nach Anspruch 32 bis 37.

Aminosäuren sind von großem wirtschaftlichen Interesse, wobei die Verwendung von Aminosäuren vielfältig ist. So wird z.B. L-Lysin wie auch L-Threonin, L-Methionin und L-Tryptophan als Futtermittelzusatz benotigt, L-Glutamat als Gewürzzusatz, L-Isoleucin und L-Tyrosin in der pharmazeutischen Industrie, L-Arginin und L-Isoleucin als Medikament oder L-Glutamat, L-Aspartat und L-Phenylalanin als Ausgangssubstanz zur Synthese von Feinchemikalien.

Eine bevorzugte Methode zur Herstellung dieser verschiedensten Aminosäuren ist die biotechnologische Herstellung mittels Mikroorganismen, denn auf diese Weise wird direkt die biologisch wirksame und optisch aktive Form der jeweiligen Aminosäure erhalten, und es können einfache und preisgünstige Rohstoffe eingesetzt werden. Als Mikroorganismen werden z.B. Corynebacterium glutamicum und seine Verwandten ssp. flavum und ssp. lactofermentum (Liebl et al., Int J System Bacteriol 1991, 41: 255 bis 260) wie auch Escherichia coli und verwandte Bakterien eingesetzt.

Diese Bakterien produzieren die Aminosäuren normalerweise aber nur in der zum Wachstum benötigten Menge, so daß also keine überschüssigen Aminosäuren gebildet und ausgeschieden werden. Dies ist darin begründet, daß in der Zelle die Biosynthese der Aminosäuren in vielfacher Weise kontrolliert wird. Folglich sind bereits verschiedenste Verfahren bekannt, um die Produktbildung durch Ausschalten der Kontrollmechanismen zu steigern. Bei diesen Prozessen werden z.B. Aminosäureanaloga eingesetzt, um die effektive Regulation der Biosynthese auszuschalten. So ist beispielsweise ein Verfahren beschrieben, bei dem Corynebacterium-Stämme benutzt werden, die gegen L-Tyrosin- und L-Phenylalaninanaloga resistent sind (JP 19037/1976 und 39517/1978). Ebenso sind Verfahren beschrieben, bei denen gegenüber L-Lysin- oder auch L-Theoninanaloga resistente Bakterien eingesetzt werden, um die Kontrollmechanismen zu überwinden (EP 0 205 849, GB 2 152 509).

Weiterhin sind auch durch rekombinante DNA-Techniken konstruierte Mikroorgansimen bekannt, bei denen ebenfalls die Regulation der Biosynthese aufgehoben ist, indem die Gene, die für die nicht mehr feedback-inhibierbaren Schlüsselenzyme kodieren, kloniert und exprimiert werden. So ist z.B. ein rekombinantes, L-Lysin produzierendes Bakterium mit plasmid-kodierter, feedback-resistenter Aspartatkinase bekannt (EP 0 381 527). Ebenso ist ein rekombinantes, L-Phenylalanin produzierendes Bakterium mit feedback-resistenter Prephenatdehydrogenase beschrieben (JP 123475/1986, EP 0 488 424).

Darüber hinaus wurden auch durch Überexpression von Genen, die nicht für feedback-sensitive Enzyme der Aminosäuresynthese kodieren, erhöhte Aminosäureausbeuten erreicht. So wird z.B. die Lysinbildung durch erhöhte Synthese der Dihydrodipicolinatsynthase verbessert (EP 0 197 335). Ebenso wird durch erhöhte Synthese der Threonindehydratase eine verbesserte Isoleucinbildung erreicht (EP 0 436 886).

Weitere Versuche zur Erhöhung der Aminosäureproduktion zielen auf eine verbesserte Bereitstellung der zellulären Primärmetabolite des Zentralstoffwechsels. So ist bekannt, daß die durch rekombinante Techniken erreichte Überexpression der Transketolase eine verbesserte Produktbildung von L-Tryptophan. L-Tyrosin oder L-Phenylalanin ermöglicht (EP 0 600 463). Weiterhin führt die Reduktion der Phosphoenolpyruvat-Carboxylase-Aktiuität in Corynebacterium zu verbesserter Bildung aromatischer Aminosäuren (EP 0 3331 145), wohingegen die Erhöhung der Phosphoenolpyruvat-Carboxylase-Aktivität in Corynebacterium zu erhöhter Ausscheidung von Aminosäuren der Aspartatfamilie führte (EP 0 358 940).

Während des Wachstums und speziell unter Aminosäureproduktionsbedingungen muß der Tricarbonsäure-Cyclus kontinuierlich und effektiv mit C4-Verbindungen, z.B. Oxalacetat, aufgefüllt werden, um die für die Aminosäurebiosynthese abgezogenen Zwischenprodukte zu ersetzen. Bis vor kurzem hat man angenommen, daß für diese sogenannten anaplerotischen Funktionen in Corynebacterium die Phosphoenolpyruvat-Carboxylase verantwortlich ist (Kinoshita, Biology of industrial micro-organisms 1985: 115 bis 142, Benjamin/Cummings Publishing Company, London; Liebl, The prokaryotes II, 1991: 1157 bis 1171, Springer Verlag N.Y.; Vallino und Stephanopoulos, Biotechnol Bioeng 1993, 41: 633 bis 646). Es wurde jedoch gefunden, daß Phosphoenolpyruvat-Carboxylase-negative Mutanten im Vergleich zu den jeweiligen Ausgangsstämmen auf allen getesteten Medien gleich wuchsen (Peters-Wendisch et al., FEMS Microbiology Leiters 1993, 112: 269 bis 274; Gubler et al., Appl Microbiol Biotechnol 1994, 40: 857 bis 863). Dieses Ergebnis zeigte, daß die Phosphoenolpyruvat-Carboxylase nicht essentiell für das Wachstum ist und für die anaplerotischen Reaktionen keine oder nur eine untergeordnete Rolle spielt. Desweiteren wies das oben genannte Ergebnis darauf hin, daß es in Corynebacterium mindestens ein anderes Enzym geben muß, das für die Synthese von Oxalacetat, das für das Wachstum benötigt wird, verantwortlich ist. Kürzlich wurde auch tatsächlich eine Pyruvat-Carboxylase-Aktivität in permeabilisierten Zellen von Corynebacterium glutamicum gefunden (Peters-Wendisch et al., Microbiology 1997, 143: 1095 bis 1103). Dieses Enzym wird effektiv durch AMP, ADP und Acetyl-Coenzym A inhibiert und in Gegenwart von Laktat als Kohlenstoffquelle in erhöhter Menge gebildet. Das Vorkommen einer Pyruvat-Carboxylase in Corynebacterium glutamicum wurde auch von einer weiteren Arbeitsgruppe mittels ¹³C-NMR Spektroskopie und GS-MS nachgewiesen bzw. bestätigt (Park et al., Appl. Microbiol. Biotechnol. 1997, 47: 430-440). Da davon ausgegangen werden mußte, daß dieses Enzym in erster Linie für die Auffüllung des Tricarbonsäure-Cycluses beim Wachstum verantwortlich ist, war zu erwarten, daß eine Erhöhung der Genexpression bzw. der Enzymaktivität entweder zu keiner oder allenfalls zu einer geringfügigen Erhöhung der zur Aspartatfamilie gehörenden Aminosäuren führt. Desweiteren wurde erwartet, daß eine Erhöhung der Genexpression bzw. der Enzymaktivität der Pyruvat-Carboxylase ebenso keinen Einfluß auf die Produktion von Aminosäuren anderer Familien haben würde.

Es wurde nunmehr überraschenderweise gefunden, daß nach Erhöhung der Pyruvat-Carboxylase-Aktivität durch genetische Veränderung des Enzyms und / oder nach Erhöhung der Pyruvat-Carboxylase-Genexpression die mikrobielle Herstellung von Aminosäuren der Aspartat- und/oder der Glutamatfamilie erhöht wird. Es zeigte sich, daß insbesondere Stämme mit erhöhter Kopienzahl des Pyruvat-Carboxylase-Gens etwa 50% mehr Lysin, 40% mehr Threonin und 150% mehr Homoserin ins Kulturmedium ausscheiden. Es zeigte sich weiterhin, daß überraschenderweise auch die Glutamatproduktion signifikant erhöht ist (vgl. insbesondere Ausführungsbeispiel unter 6. und Tabelle 4).

Die genetische Veränderung der Pyruvat-Carboxylase zur Erhöhung der Enzymaktivität erfolgt vorzugsweise durch Mutation des endogenen Gens. Derartige Mutationen können entweder nach klassischen Methoden ungerichtet erzeugt werden, wie beispielsweise durch UV-Bestrahlung oder durch mutationsauslösenden Chemikalien, oder gezielt mittels gentechnologischer Methoden wie Deletion(en), Insertion(en) und/oder Nukleotidaustausch(e).

Die Pyruvat-Carboxylase-Genexpression wird durch Erhöhen der Genkopienzahl und/oder durch Verstärkung regulatorischer Faktoren, die die Expression des Gens positiv beeinflussen, erhöht. So kann eine Verstärkung regulatorischer Elemente vorzugsweise auf der Transkriptionsebene erfolgen, indem insbesondere die Transkriptionssignale erhöht werden. Dies kann beispielsweise dadurch erfolgen, daß durch Veränderung der dem Strukturgen vorgeschalteten Promotorsequenz der Promotor in seiner Wirksamkeit erhöht wird oder indem der Promotor komplett durch wirksamere Promotoren ausgetauscht wird Auch kann eine Verstärkung der Transkription durch entsprechende Beeinflussung eines dem Pyruvat-Carboxylase-Gens zugeordneten Regulatorgens erfolgen. Desweitern kann ggf durch Mutation einer regulatorischen Gensequenz die Effektivitat der Bindung eines Regulatorporteins an die DNA des zu regulierenden Pyruvat-Carboxylase-Gens so beeinflußt sein, daß dadurch die Transkription verstärkt und somit die Genexpression erhöht ist. Desweiteren können dem Pyruvat-Carboxylase-Gen als regulatorische Sequenzen aber auch sog. "enhancer" zugeordnet sein, die über eine verbesserte Wechselwirkung zwischen RNA-Polymerase und DNA ebenfalls eine erhöhte Pyruvat-Carboxylase-Genexpression bewirken. Daneben ist aber auch eine Verstärkung der Translation möglich, indem beispielsweise die Stabilität der m-RNA verbessert wird.

Zur Erhöhung der Genkopienzahl wird das Pyruvat-Carboxylase-Gen in ein Genkonstrukt bzw. Vektor eingebaut. Das Genkonstrukt enthält insbesondere dem Pyruvat-Carboxylase-Gen zugeordnete regulatorische Sequenzen, vorzugsweise solche, die die Genexpression verstärken. Für den Einbau des Pyruvat-Carboxylase-Gens in ein Genkonstrukt wird das Gen vorzugsweise aus einem Mikroorganismen-Stamm der Gattung Corynebacterium isoliert und in einen Aminosäure-produzierenden Mikroorganismen-Stamm, insbesondere Corynebacterium oder in Escherichia coli oder Serratia marcescens. transformiert. Für das erfindungsgemäße Verfahren eignen sich insbesondere Gene aus C. glutamicum oder C glutamicum ssp. flavum oder C. glutamicum ssp. lactofermentum. Nach Isolierung des Gens und der in vitro-Rekombination mit bekannten Vektoren (vgl. z.B. Simon et al., Bio/Technology 1983, 1: 784 bis 791; Eikmanns et al., Gene 1991, 102: 93 bis 98) erfolgt die Transformation in die Aminosäure-produzierenden Stämme durch Elektroporation (Liebl et al., FEMS Microbiology Leiters 1991, 65: 299 bis 304) oder Konjugation (Schäfer et al., J Bacteriol 1990, 172: 1663 bis 1666). Als Wirtsstämme werden vorzugsweise solche Aminosäureproduzenten eingesetzt, die in der Synthese der entsprechenden Aminosäure dereguliert sind und/oder die eine erhöhte Exportcarrier-Aktivität für die entsprechende Aminosäure aufweisen. Weiterhin werden solche Stämme bevorzugt, die einen erhöhten Anteil an solchen Zentralstoffwechselmetaboliten enthalten, die an der Synthese der entsprechenden Aminosäure beteiligt sind und / oder Stämme, die einen erniedrigten Anteil an den nicht an der Synthese der entsprechenden Aminosäure beteiligten Zentralstoffwechselmetaboliten enthalten, insbesondere an Metaboliten, die für Konkurrenzreaktionen zuständig sind; d.h. es werden solche Stämme bevorzugt, bei denen ein zu dem entsprechenden Aminosäurebiosyntheseweg konkurrierender Biosyntheseweg mit verminderter Aktivität abläuft. So ist insbesondere ein, gegen L-Asparaginsäure-β-Methylester (AME) resistenter coryneformer Mikroorganismen-Stamm mit reduzierter Citrat-Synthase-Aktivität geeignet (EP 0 551 614).

Nach Isolierung sind Pyruvat-Carboxylase-Gene mit Nukleotidsequenzen erhältlich, die für die unter SEQ ID No. 2 angegebenen Aminosäuresequenz oder deren Allelvariationen kodieren bzw. die die Nukleotidsequenz von Nukleotid 165 bis 3587 gemäß SEQ ID No. 1 oder einer im wesentlichen gleichwirkenden DNA-Sequenz aufweisen. Desweiteren sind Gene mit einem vorgeschalteten Promotor der Nukleotidsequenz von Nukleotid 20 bis 109 gemäß SEQ ID No. 1 oder eine im wesentlichen gleichwirkende DNA-Sequenz erhältlich. Allelvariationen bzw. gleichwirkende DNA-Sequenzen umfassen insbesondere funktionelle Derivate. die durch Defetion(en), Insertion(en) und/oder Substitution(en) von Nukleotiden aus entsprechenden Sequenzen erhältlich sind, wobei die Enzymaktivität bzw. -funktion erhalten bleibt oder sogar erhöht ist. Diese Pyruvat-Carboxylase-Gene werden vorzugsweise im erfindungsgemäßen Verfahren eingesetzt.

Dem Pyruvat-Carboxylase-Gen mit oder ohne vorgeschaltetem Promotor bzw. mit oder ohne zugeordnetem Regulatorgen können ein oder mehrere DNA-Sequenzen vor- und/oder nachgeschaltet sein, so daß das Gen in einer Genstruktur enthalten ist.

Dem Pyruvat-Carboxylase-Gen ist vorzugsweise der tac-Promotor (lacI^{Q}-Gen) vorgeschaltet, wobei diesem insbesondere regulatorische Sequenzen zugeordnet sind.

Durch Klonierung des Pyruvat-Carboxylase-Gens sind Plasmide erhältlich, die das Gen enthalten und zur Transformation eines Aminosäureproduzenten geeignet sind. Die durch Transformation erhältlichen Zellen, bei denen es sich vorzugsweise um transformierte Zellen von Corynebacterium handelt, enthalten das Gen in replizierbarer Form, d.h. in zusätzlichen Kopien auf dem Chromosom, wobei die Genkopien durch Rekombination an beliebigen Stellen des Genoms integriert werden, und/oder auf einem Plasmid oder Vektor.

### Ausführungsbeispiel

### 1. Klonierung des Pyruvat-Carboxylase-Gens aus Corynebacterium glutamicum

Ausgehend von konservierten Bereichen aller bisher bekannten Pyruvat-Carboxylase-(pyc-)Genen, von Saccharomyces cerevisiae (J Biol Chem 1988, 263: 11493-11497; Mol Gen Genet 1991, 229: 307-315), Mensch (Biochim Biophys Acta 1994, 1227: 46-52). Maus (Proc Natl Acad Sci, USA 1993, 90: 1766-1770), Aedes aegypti (EMBL-GenBank: Accession Nr. L36530) sowie von Mycobacterium tubercolosis (EMBL-GenBank: Accession Nr. U00024) wurden PCR-Primer synthetisiert (MWG Biotech). Die Primer entsprachen den Basen 810 bis 831 und 1015 bis 1037 des pyc-Gens von M. tuberculosis. Mit diesen Primern konnte mittels PCR nach der Standardmethode von Innis et al. (PCR protocols. A guide to methods and applications, 1990, Academic Press) für nicht-degenerierte, homolge Primer ein Fragment von ca. 200 bp aus chromosomaler DNA von C. glutamicum ATCC 13032. die wie bei Eikmanns et al. (Microbiology 1994, 140: 1817-1828) beschrieben, isoliert wurde, amplifiziert werden. Die Größe von 200 bp entsprach der Erwartung für pyc-Gene. Das PCR-Produkt wurde wie bei Sanger et al. (Proc Natl Acad Sci USA 1977, 74: 5463-5467) beschrieben, sequenziert. Die Sequenzierung wurde mit fluoreszenzmarkierten ddNTPs mit einer automatischen DNA-Sequenzierapparatur (Applied Biosystems) durchgeführt.

Ausgehend von diesem DNA-Fragment aus C. glutamicum wurden folgende homologe Oligonukleotide hergestellt:

Die Oligonukleotide wurden als PCR-Primer zur Isolierung einer Sonde für das Gen der Pyruvat-Carboxylase (pyc) aus C. glutamicum verwendet. Die Primer wurden in eine PCR-Reaktion mit chromosomaler DNA von C. glutamicum und Digoxigeninmarkierten Nukleotiden eingesetzt. Die Reaktion wurde nach der Vorschrift des 'PCR DIG Labeling Kits' der Firma Boehringer Mannheim durchgerührt. Mit diesem Ansatz konnte ein Digoxigenin-markiertes DNA-Fragment amplifiziert werden, das der erwarteten Größe von ca. 200 bp entsprach. Die so hergestellte pyc-Sonde wurde dann eingesetzt, um über Southern-Blot-Hybridisierung ein DNA-Fragment in der chromosomalen DNA von C. glutamicum zu identifizieren, auf dem das pyc-Gen lokalisiert ist. Hierzu wurden jeweils 2 bis 5 µg chromosomaler DNA von C. glutamicum WT mit den Restriktionsenzymen HindIII, SphI, SalI, DraI, EcoRI und BamHI geschnitten, die erhaltenen DNA-Fragmente 16 h bei 20 V in einem 0,8 %igen Agarosegel gelelektroploretisch ihrer Größe entsprechend aufgetrennt. Die in dem Agarosegel befindlichen DNA-Fragmente wurden nach einer Methode von Southern (J Mol Biol 1975, 98: 503-517) denaturiert und vakuumunterstützt mit der VacuGene Blot Apparatur von Pharmacia LKB (Uppsala, Schweden) aus der Gelmatrix auf eine Nylon-Membran (Nytran N 13 von Schleicher und Schüll, Dassel, Schweiz) transferiert, immobilisiert und die Digoxigeninmarkierung mittels NBT/X-Phosphat-Umsetzung durch alkalische Phosphatase nachgewiesen. Auf diese Weise konnten folgende, mit der pyc-DNA-Sonde hybridisierende chromosomale Fragmente nachgewiesen werden: ein 17 kb HindIII-Fragment, ein 6,5 kb SalI-Fragment und ein 1,35 kb EcoRI-Fragment.

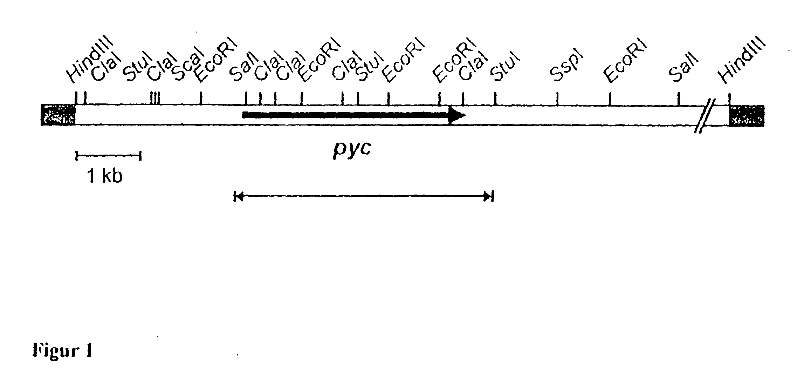

Das 17 kb HindIII-Fragment wurde isoliert und subkloniert. Dazu wurde eine Cosmid-Genbank aus chromosomaler DNA von C. glutamicum im Cosmid pHC79 verwendet, die das Genom von C. glutamicum zu 99% repräsentierte (Mol Microbiol 1992, 6: 317-326). Der E. coli-Stamm DH5α wurde mit dieser Genbank mittels der CaCl₂-Methode von Sambrook et al. (Molecular Cloning, A laboratory manual, 1989, Cold Spring Habour Laboratory Press) transformiert und zu ca. 300 Kolonien pro LB-Agarplatte mit 50 µg/l Kanamycin ausplattiert (insgesamt 5000 Kolonien). Anschließend wurden die erhaltenen Transformanden auf Nytran N13-Filter übertragen und diese zur alkalischen Lyse der Zellen und Denaturierung der DNA auf mit 0,5 M NaOH und 1,5 M NaCl getränktem Whatmann-Papier 5 min, inkubiert. Die darauffolgende Neutralisierung erfolgte mit 1 M Tris/HCl pH 7,5 und 1,5 M NaCl. Nach Inkubation der Filter in 2 x SSC wurde die freigesetzte DNA durch UV-Bestrahlung bei 366 nm auf dem Filter fixiert. Anschließend wurden die restlichen Zelltrümmer durch Schütteln in 3 x SSC, 0,1 % SDS bei 50°C entfernt. Die Filter wurden in dieser Form für die Hybridisierung mit einer spezifischen pyc-Sonde, wie bei Southern (J Miol Biol 1975, 98: 503-517) beschrieben, verwendet. Es wurden 3 Transformanden identifiziert, die gegen die pyc-Sonde hybridisierten. Aus diesen Transformanden wurde die Cosmid-DNA mittels Plasmid-Präparation nach der Methode der alkalischen Lyse von Birnboim (Meth Enzymol 1983, 100: 243-255) isoliert und anschließend über Restriktion und Southern-Blot Analyse auf das Vorhandensein des HindIII-Fragments getestet. Das Cosmid pHC79-10, das ein 40 kb Insert enthielt, trug das 17 kb HindIII-Fragment vollständig und wurde weiter analysiert. Es zeigte sich, daß auch nach Restriktion mit den Endonukleasen SalI und EcoRI die gleichen hybridisierenden Fragmente wie in der chromosomalen DNA, d.h. ein 6,5 kb Sall- und ein 1,35 kb EcRI-Fragment, erhalten wurden Das 17 kb HindIII-Fragment wurde durch Restriktion mit HindIII aus dem Cosmid isoliert und in den E. coli-Vektor pUC18, der ebenfalls mit HindIII geschnitten wurde, ligiert. Es wurde eine Restriktionsanalyse des Fragments in dem resultierenden Vektor pUCpyc erstellt. Die physikalische Kartierung des Fragments ist in Figur 1 dargestellt,

### 2. Sequenzierung des Pyruvat-Carboxylase-Gens

In weiteren Subklonierungsschritten wurden ein 0,85 kb SalI-EcoRI-Fragment, das 1,35 kb EcoRI-Fragment, ein 1,6 kb EcoRI-EcoRI-StuI-Fragment sowie ein 1,6 kb ClaI-Fragment, das partiell mit dem 0,85 kb SalI-EcoRI-Fragment überlappte, durch Restriktion mit den entsprechenden Restriktionsenzymen aus dem Plasmid pUCpyc isoliert. Durch Ligation wurden die Fragmente in den jeweils entsprechend restringierten Vektor pUC18 kloniert und anschließend nach Sanger et al. (Proc Nati Acad Sci USA 1977, 74: 5463-5467) wie oben beschrieben sequenziert. Die erhaltenen Nukleotidsequenzen wurden mit dem Programmpaket HUSAR (Release 3.0) des Deutschen Krebsforschungszentrums (Heidelberg) analysiert. Die Sequenzanalyse der Fragmente ergab ein durchgehendes offenes Leseraster von 3576 bp, das für eine Proteinsequenz von 1140 Aminosäuren kodiert. Ein Vergleich der abgeleiteten Proteinsequenz mit der EMBL Gen-Datenbank (Heidelberg) ergab Ähnlichkeiten zu allen bekannten Pyruvat-Carboxylasen. Die höchste Identitat (62%) wurde zur putativen Pyruvat-Carboxylase aus Mycobacterium tuberculosis (EMBL-GenBank: Accession Nr. U00024) gefunden. Die Ähnlichkeit betrug, unter Berücksichtigung konservierter Aminosäureaustausche, 76%. Ein Vergleich mit den Pyruvat-Carboxylasen anderer Organismen ergab 46 bis 47% identische und 64 bis 65% ähnliche Aminosäuren (Gene 1997, 191: 47-50; J Bacteriol 1996, 178: 5960-5970; Proc Natl Acad Sci USA 1993, 90: 1766-1770; Biochem J 1996, 316: 631-637; EMBL-GenBank: Accession Nr. L36530; J Biol Chem 1988, 263: 11493-11497; Mol Gen Genet 1991, 229: 307-315). Aus diesen Ergebnissen wurde geschlossen, daß das klonierte Fragment das Gen für die Pyruvat-Carboxylase aus C. glutamicum trägt. Die Nukleotidsequenz des Gens ist unter SEQ ID No.1 und die entsprechende Aminosäuresequenz unter SEQ ID No. 2 angegeben.

### 3. Überexpression des Pyruvat-Carboxylase-Gens

Zur Überexpression des Gens für die Pyruvat-Carboxylase aus C. glutamicum wurde das Gen aus dem Plasmid pUCpyc als 6,2 kb SspI-ScaI-Fragment in den E. coli-C. Glutamicum-Pendelvektor pEKO (Gene 1991, 102: 93-98) kloniert, der mit den Restriktionsendonukleasen EcoRI und PstI geschnitten wurde. Mittels Klenow-Polymerase-Behandlung wurden die überhängenden Enden zu glatten Enden aufgefüllt (EcoRI) bzw. abgedaut (PstI), und der linearisierte Vektor wurde mit dem 6,2 kb SspI-ScaI-Fragment ligiert. Das erhaltene Konstrukt pEK0pyc wurde zunächst in den Stamm E. coli DH5α transformiert, die Plasmid-DNA auf den erhaltenen Transformanden isoliert und auf die Richtigkeit des Inserts durch Restriktion kontrolliert. Die DNA wurde anschließend in den Stamm SP733 durch Elektroporation eingebracht (FEMS Microbiol Lett 1989, 65: 299-304). Bei diesem Stamm handelt es sich um eine Mutante des restriktionsnegativen C. glutamicum Stammes R127 (Dechema Biotechnology Conference 1990, 4: 323-327, Verlag Chemie), die durch chemische Mutagenese erhalten worden war und sich dadurch auszeichnet, daß sie nicht auf Minimalmedium mit Pyruvat und Lactat als einziger Kohlenstoffquelle wachsen kann (Microbiology 1997, 143: 1095-1103). Dieser Phänotyp wird durch einen Defekt in der Pyruvat-Carboxylase hervorgerufen und konnte durch das Einbringen des Pyruvat-Carboxylase-Gens aus C. glutamicum komplementiert werden, d.h. der Stamm, der das Plasmid pEK0pyc trägt, war im Gegensatz zum Ausgangsstamm wieder in der Lage auf Minimalmedium mit Lactat als einziger Kohlenstoffquelle zu wachsen. Damit war auch der Beweis erbracht, daß das Gen für eine funktionelle Pyruvat-Carboxylase kodiert.

Darüber hinaus wurde das Plasmid pEK0pyc in den C. glutamicum Wildtyp ATCC 13032 durch Elektroporation transformiert. Der resultierende Stamm WT (pEK0pyc) wurde im Vergleich zum Wildtyp ATCC 13032 bezüglich seiner Pyruvat-Carboxylase-Aktivität untersucht. Die Stämme wurden in Komplexmedium (Luria-Bertani, Molecular Cloning, A laboratory manual, 1989, Cold Spring Harbour Laboratory Press) mit 0,5 % Lactat und auf Minimalmedium mit 2 % Lactat bzw. 4 % Glukose gezüchtet, und der Pyruvat-Carboxylase-Test wurde entsprechend der Methode, wie sie bei Peters-Wendisch et al (Microbiology 1997, 143: 1095-1103) beschrieben wurde, durchgeführt Das Ergebnis der Analyse (Tabelle 1) zeigt, daß die Pyruvat-Carboxylase-Aktivität im pEK0-pyc-tragenden Stamm ca. 4-fach höher als im Ausgangsstamm war.

### 4. Gesteigerte Akkumulation von Lysin durch Überexpression des Pyruvat-Carboxylase-Gens im Stamm C. glutamicum DG52-5

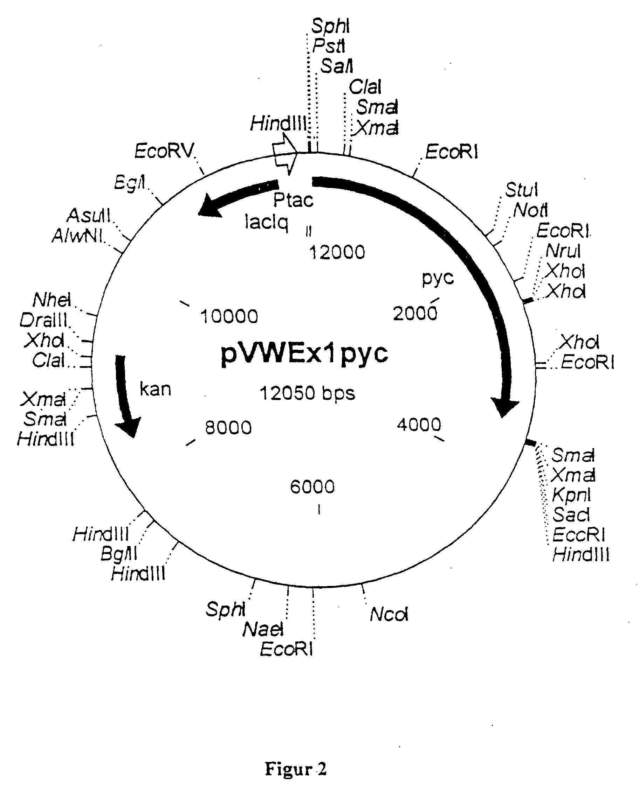

Zur Untersuchung der Auswirkung der Überexpression des Gens für die Pyruvat-Carboxylase in dem Lysin-Produktionsstamm DG52-5 (J Gen Microbiol 1988, 134: 3221-3229) wurde der Expressionsvektor pVWEX1 verwendet, der eine IPTGinduzierbare Expression erlaubt. In diesen Vektor wurde das pyc Gen promotorlos hinein kloniert. Dazu wurden zunächst PCR-Primer (Primer 1 = Position 112 - 133; Primer 2 = Position 373 bis 355 in der Nukleotidsequenz gemäß SEQ ID No. 1) synthetisiert und 261 bp des promotorlosen Anfangsbereichs des Pyruvat-Carboxylase-Gens mittels PCR amplifiziert. Die Primer wurden so gewählt, daß Primer 1 eine Pstl-Schnittstelle vermittelt und Primer 2 eine BamHI-Schnittstelle, Nach der PCR wurde das erhaltene 274 bp PCR-Produkt isoliert, zu Konkatemeren ligiert und anschließend mit den Restriktionsenzymen Pstl und BamHI geschnitten. Der Restriktionsansatz wurde durch Ethanol-Fällung ankonzentriert und anschließend mit dem PstI-BamHI-geschnittenen Vektor pVWEX1 ligiert. Das erhaltene Konstrukt pVWEX1-PCR wurde durch Restriktion getestet. Der Endbereich des pyc Gens wurde durch RcaI-Klenow-SalI-Behandlung aus dem Vektor pEK0pyc isoliert und in den BamHI-Klenow-SalI behandelten Vektor pVWEX1-PCR ligiert. Das erhaltene Konstrukt pVWEX1pyc wurde durch Restriktionskartierung analysiert. Eine physikalische Karte des Plasmids ist in Figur 2 gezeigt.

Das Plasmid wurde durch Elektroporation in den C. glutamicum Stamm DG52-5 eingebracht. Als Kontrolle wurde der Stamm DG52-5 mit dem Vektor pVWEX1 ohne Insert transformiert und die L-Lysinausscheidung jeweils drei verschiedener Transformanden verglichen. Dazu wurden DG52-5(pVWEX1)1, 2 und 3 sowie DG52-5(pVWEX1pyc)3, 4 und 6 in Komplexmedium (2xTY; Molecular Cloning, A laboratory manual, 1989, Cold Spring Harbour Laboratory Press; mit 50 µg/l Kanamycin) gezüchtet und das Fermentationsmedium CGXII (J Bacteriol 1993, 175: 5595-5603) jeweils aus den Vorkulturen getrennt beimpft. Das Medium enthielt zusätzlich Kanamycin, um die Plasmide stabil zu halten. Es wurden jeweils zwei parallele Ansätze durchgeführt, wobei einem Kolben 200 µg IPTG/ml zugesetzt wurde, während der zweite Kolben kein IPTG enthielt. Nach Kultivierung für 48 Stunden bei 30°C auf dem Rotationsschüttler bei 120 Upm wurde die in das Medium akkumulierte Lysinmenge bestimmt. Die Bestimmung der Aminosäurekonzentration erfolgte mittels Hochdruckflüssigkeitschromatographie (J Chromat 1983, 266: 471-482). Das Ergebnis der Fermentation ist in Tabelle 2 dargestellt, wobei die angegebenen Werte Mittelwerte aus jeweils drei Experimenten mit unterschiedlichen Klonen darstellen. Es zeigte sich, daß die Überexpression des Pyruvat-Carboxylase-Gens zu einer um 50 % gesteigerten Akkumulation von Lysin im Medium führt. Somit stellt die Nutzung des entdeckten und beschriebenen Gens für das anaplerotische Enzym Pyruvat-Carboxylase ein Verfahren dar, um die L-Lysinbildung entscheidend zu verbessern.

### 5. Gesteigerte Akkumulation von Threonin und Homoserin durch Überexpression des Pyruvat-Carboxylase-Gens im Stamm C. glutamicum DM368-3

Analog zu den Experimenten zur L-Lysin-Bildung wurde auch die Akkumulation von Threonin im Kulturüberstand durch Überexpression des Gens für die Pyruvat-Carboxylase untersucht. Hierzu wurde, wie unter Punkt 4 beschrieben, der Threoninproduktionsstamm C. glutamicum DM368-3 (Degussa AG) mit dem Plasmid pVWEX1pyc sowie zur Kontrolle mit dem Plasmid pVWEX1 transformiert und die Threoninausscheidung von jeweils drei verschiedenen Transformanden untersucht. Dazu wurden DM368-3(pVWEX1)1, 2 und 3 sowie DM368-3(pVWEX1pyc)1, 2 und 3 in Komplexmedium (2xTY mit 50 µg/l Kanamycin) gezüchtet und das Fermentationsmedium CGXII (J Bacteriol 1993, 175: 5595-5603) jeweils aus den Vorkulturen getrennt beimpft. Das Medium enthielt zusatzlich Kanamycin, um die Plasmide stabil zu halten. Es wurden zwei parallele Ansätze durchgeführt, wobei einem Kolben 200 µg IPTG/ml zugesetzt wurde, während der zweite Kolben kein IPTG enthielt. Nach Kultivierung für 48 Stunden bei 30°C auf dem Rotationsschüttler bei 120 Upm wurde die in das Medium akkumulierte Threoninmenge bestimmt. Die Bestimmung der Aminosäurekonzentration erfolgte ebenfalls mittels HochdruckFlüssigkeitschromatographie (J Chromat 1983, 266: 471-482). Das Ergebnis der Fermentation ist in Tabelle 3 dargestellt, wobei die angegebenen Werte Mittelwerte aus jeweils drei Experimenten mit unterschiedlichen Klonen darstellen. Es zeigte sich, daß die Überexpression des Pyruvat-Carboxylase-Gens zu einer ca. 40%igen Steigerung der Threoninkonzentration im Medium führt. Somit stell die Nützung des endeckten und beschriebenen Gens für das anaplerotische Enzym Pyruvat-Carboxylase ein Verfahren dar, um die L-Threoninbildung entscheidend zu verbessern. Desweiteren zeigte die Aminosäurekonzentrationsbestimmung, daß überraschenderweise der Stamm mit überexprimiertem Pyruvat-Carboxylase-Gen außerdem etwa 150% mehr Homoserin ins Medium ausschied als der Stamm mit nicht überexprimiertem Gen. Die entsprechenden Ergebnisse sind ebenfalls in Tabelle 3 dargestellt. Sie machen deutlich, daß durch das erfindungsgemäße Verfahren sowohl die Threonin- als auch die Homoserinbildung entscheidend verbessert werden kann.

### 6. Gesteigerte Akkumulation von Glutamat durch Überexpression des Gens für die Pyruvat-Carboxylase im Wildtyp von C. glutamicum

Analog zu den Experimenten zur L-Lysin-, L-Threonin- und L-Homoserin-Bildung (siehe oben unter 4. und 5.) wurde auch die Akkumulation von Glutamat im Kulturüberstand durch Überexpression des Gens für die Pyruvat-Carboxylase untersucht. Hierzu wurde, wie unter Punkt 4 beschrieben, der Wildtyp C. glutamicum ATCC 13032 mit dem Plasmid pVWEX1pyc sowie zur Kontrolle mit dem Plasmid pVWEX1 transformiert und die Glutamatausscheidung von jeweils zwei verschiedenen Transformanden untersucht. Dazu wurde C. glutamicum ATCC 13032 (pVWEX1pyc) D1 und D2 sowie C. glutamicum ATCC 13032 (pVWEX1 pyc) 1 und 2 in Komplexmedium (2xTY mit 50 µg/l Kanamycin) gezüchtet und das Fermentationsmedium CGXII (J Bacteriol 1993, 175: 5595-5603) jeweils aus den Vorkulturen getrennt beimpft. Das Medium enthielt zusätzlich Kanamycin, um die Plasmide stabil zu halten. Zur Induktion der Glutamatausscheidung wurde dem Medium ca. 6 Stunden nach dem Inokulieren 25 mg Tween 60 pro ml zugesetzt. Es wurden zwei parallele Ansätze durchgeführt, wobei einem Kolben 200 µg IPTG/ml zugesetzt wurde, während der zweite Kolben kein IPTG enthielt. Nach Kultivierung für 48 Stunden bei 30°C auf dem Rotationsschüttler bei 120 Upm wurde die in das Medium akkumulierte Glutamatmenge bestimmt. Die Bestimmung der Aminosäurekonzentration erfolgte ebenfalls mittels HochdruckFlüssigkeitschromatographie (J Chromat 1983, 266: 471-482). Das Ergebnis der Fermentation ist in Tabelle 4 dargestellt, wobei die angegebenen Werte Mittelwerte aus jeweils zwei Experimenten mit unterschiedlichen Klonen darstellen. Es zeigte sich, daß die Überexpression des Pyruvat-Carboxylase-Gens zu einer bis zu 500%igen Steigerung der Glutamatkonzentration im Medium führt. Somit stell die Nutzung des endeckten und beschriebenen Gens für das anaplerotische Enzym Pyruvat-Carboxylase ein Verfahren dar, um die L-Glutamatbildung entscheidend zu verbessern.

**Tabelle 1**

| Stamm | IPTG [µg/ml] | Pyruvat-Carboxylase [nmol min⁻¹ mg Trockengewicht⁻¹] |
|---|---|---|
| 13032(pEK0pyc) | 0 | 75 ± 13 |
| ATCC 13032 | 0 | 19 ± 4 |
| DG52-5(pVWEX1pyc) | 200 | 88 ± 13 |
| | 0 | 11 ± 2 |
| DG52-5(pVWEX1) | 200 | 5 ± 2 |
| | 0 | 6 ± 1 |
| DM368-3(pVWEX1pyc) | 200 | 76 ± 10 |
| | 0 | 12 ± 3 |
| DM368-3(pVWEX1) | 200 | 10 ± 1 |
| | 0 | 11 ± 2 |

**Tabelle 2**

| Stamm | IPTG [µg/ml] | Lysin [mM] |
|---|---|---|
| DG52-5(pVWEX1pyc) | 200 | 35,4±2,6 |
| | 0 | 23,6 ± 2,9 |
| DG52-5(pVWEX1) | 200 | 23,3 ± 2,9 |
| | 0 | 22,1 ± 4,0 |

**Tabelle 3**

| Stamm | IPTG [µg/ml] | Threonin [mM] | Homoserin [mM] |
|---|---|---|---|
| DM368-3(pVWEX1 pyc) | 200 | 10,2 ± 0,5 | 14,4 ± 1,2 |
| | 0 | 7,9 ± 1,0 | 5,6 ± 0,2 |
| DM368-3(pVWEX1) | 200 | 8,0 ± 0,5 | 5,8 ± 0,7 |
| | 0 | 7,5 ± 0,8 | 6,1 ± 1,0 |

**Tabelle 4**

| Stamm | IPTG [µg/ml] | Glutamat [mM] |
|---|---|---|
| ATCC 13032 | 200 | 11 ± 2 |
| ATCC 13032 | 0 | 13 ± 2 |
| ATCC 13032(pVWEX1-pyc) | 200 | 67 ± 4 |
| ATCC 13032(pVWEX1-pyc) | 0 | 32 ± 4 |

## Patentansprüche

1. Verfahren zur mikrobiellen Herstellung von Aminosäuren der Aspartatund/oder Glutamatfamilie, bei dem die Pyruvat-Carboxylase-Aktivität durch genetische Veränderung des Enzyms und / oder die Pyruvat-Carboxylase-Genexpression eines die entsprechende Aminosäure produzierenden Mikroorganismus erhöht wird.

2. Verfahren nach Anspruch 1,
**dadurch gekennzeichnet,**
**daß** durch Mutation des endogenen Pyruvat-Carborylase-Gens ein Enzym mit höherer Pyruvat-Carboxylase-Aktivität erzeugt wird.

3. Verfahren nach Anspruch 1 oder 2,
**dadurch gekennzeichnet,**
**daß** die Genexpression der Pyruvat-Carboxylase durch Erhöhen der Genkopienzahl erhöht wird.

4. Verfahren nach Anspruch 3,
**dadurch gekennzeichnet,**
**daß** zur Erhöhung der Genkopienzahl das Pyruvat-Carboxylase-Gen in ein Genkonstrukt eingebaut wird.

5. Verfahren nach Anspruch 4,
**dadurch gekennzeichnet,**
**daß** das Gen in ein Genkonstrukt eingebaut wird, das dem Pyruvat-Carboxylase-Gen zugeordnete regulatorische Gensequenzen enthält.

6. Verfahren nach Anspruch 4 oder 5,
**dadurch gekennzeichnet,**
**daß** ein die entsprechende Aminosäure produzierender Mikroorganismus mit dem das Gen enthaltende Genkonstrukt transformiert wird.

7. Verfahren nach Anspruch 6,
**dadurch gekennzeichnet,**
**daß** ein Mikroorganismus der Gattung Corynebacterium mit dem das Gen enthaltende Genkonstrukt transformiert wird.

8. Verfahren nach Anspruch 6 oder 7,
**dadurch gekennzeichnet,**
**daß** für die Transformation ein Mikroorganismus eingesetzt wird, in dem die an der Synthese der entsprechenden Aminosäure beteiligten Enzyme dereguliert sind und / oder die eine erhöhte Exportcarrier-Aktivität für die entsprechende Aminosäure aufweisen.

9. Verfahren nach einem der Ansprüche 6 bis 8,
**dadurch gekennzeichnet,**
**daß** für die Transformation ein Mikroorganismus eingesetzt wird, der einen erhöhten Anteil an den an der Synthese der entsprechenden Aminosäure beteiligten Zentralstoffwechselmetaboliten enthält.

10. Verfahren nach einem der Ansprüche 6 bis 9,
**dadurch gekennzeichnet,**
**daß** für die Transformation ein Mikroorganismus eingesetzt wird, bei dem ein zu dem entsprechenden Aminosäurebiosyntheseweg konkurrierender Biosyntheseweg mit verminderter Aktivität abläuft.

11. Verfahren nach einem der vorhergehenden Ansprüche,
**dadurch gekennzeichnet,**
**daß** das Pyruvat-Carboxylase-Gen aus einem Mikroorganismus-Stamm der Gattung Corynebacterium isoliert wird.

12. Verfahren nach einem der vorhergehenden Ansprüche,
**dadurch gekennzeichnet,**
**daß** die Genexpression durch Verstärkung der Transkriptionssignale erhöht wird.

13. Verfahren nach einem der vorhergehenden Ansprüche,
**dadurch gekennzeichnet,**
**daß** dem Pyruvat-Carboxylase-Gen der tac-Promotor vorgeschaltet wird.

14. Verfahren nach Anspruch 13,
**gekennzeichnet durch**
dem tac-Promotor zugeordnete regulatorische Sequenzen.

15. Verfahren nach einem der vorhergehenden Ansprüche,
**dadurch gekennzeichnet,**
**daß** als Pyruvat-Carboxylase-Gen ein Gen mit einer für die unter SEQ ID No. 2 angegebenen Aminosäuresequenz und deren Allelvariationen kodierenden Nukleotidsequenz eingesetzt wird.

16. Verfahren nach Anspruch 15,
**dadurch gekennzeichnet,**
**daß** als Pyruvat-Carboxylase-Gen ein Gen mit der Nukleotidsequenz von Nukleotid 165 bis 3587 gemäß SEQ ID No. 1 oder einer im wesentlichen gleichwirkenden DNA-Sequenz eingesetzt wird.

17. Verfahren nach einem der vorhergehenden Ansprüche zur Herstellung von Lysin, Threonin, Homoserin, Glutamat und/oder Arginin.

18. Pyruvat-Carboxylase-Gen mit einer für die unter SEQ ID No. 2 angegebenen Aminosäuresequenz und / oder deren Allelvariationen kodierenden Nukleotidsequenz.

19. Pyruvat-Carboxylase-Gen nach Anspruch 18 mit der Nukleotidsequenz von Nukleotid 165 bis 3587 gemäß SEQ ID Nr. 1 oder einer im wesentlichen gleichwirkenden DNA-Sequenz.

20. Pyruvat-Carboxylase-Gen nach Anspruch 18 oder 19 mit einem vorgeschalteten Promotor der Nukleotidsequenz von Nukleotid 20 bis 109 gemäß SEQ ID No. 1 oder einer im wesentlichen gleichwirkenden DNA-Sequenz.

21. Pyruvat-Carboxylase-Gen nach Anspruch 18 oder 19 mit vorgeschaltetem tac-Promotor

22. Pyruvat-Carboxylase-Gen nach Anspruch 21 mit dem Promotor zugeordneten regulatorischen Sequenzen.

23. Pyruvat-Carboxylase-Gen nach einem der Ansprüche 18 bis 20 mit diesem zugeordnete regulatorische Gensequenzen.

24. Genstruktur, enthaltend ein Pyruvat-Carboxylase-Gen nach einem der Ansprüche 18 bis 23.

25. Vektor, enthaltend ein Pyruvat-Carboxylase-Gen nach einem der Ansprüche 18 bis 23 oder eine Genstruktur nach Anspruch 24.

26. Transformierte Zelle, enthaltend in replizierbarer Form ein Pyruvat-Carboxylase-Gen nach einem der Ansprüche 18 bis 23 oder eine Genstruktur nach Anspruch 24.

27. Transformierte Zelle nach Anspruch 26, enthaltend einen Vektor nach Anspruch 25.

28. Transformierte Zelle nach Anspruch 26 oder 27,
**dadurch gekennzeichnet,**
**daß** sie der Gattung Corynebacterium angehört.

29. Transformierte Zelle nach einem der Ansprüche 26 bis 28,
**dadurch gekennzeichnet,**
**daß** in dieser die an der Synthese der entsprechenden Aminosäure beteiligten Enzyme und / oder die am Export der entsprechenden Aminosäure beteiligten Enzyme dereguliert sind.

30. Transformierte Zelle nach einem der Ansprüche 26 bis 29,
**dadurch gekennzeichnet,**
**daß** sie einen erhöhten Anteil an den an der Synthese der entsprechenden Aminosäure beteiligten Zentralstoffwechselmetaboliten enthält.

31. Transformierte Zelle nach einem der Ansprüche 26 bis 30,
**dadurch gekennzeichnet,**
**daß** sie einen erniedrigten Anteil an den nicht an der Synthese der entsprechenden Aminosäure beteiligten Zentralstoffwechselmetaboliten enthält.

32. Verwendung eines Pyruvat-Carboxylase-Gens zur Steigerung der Produktion von aus der Aspartat- und/oder Glutamatfamilie stammenden Aminosäuren von Mikroorganismen.

33. Verwendung nach Anspruch 32,
**dadurch gekennzeichnet,**
**daß** ein mutiertes Pyruvat-Carboxylase-Gen, das für ein Enzym mit erhöhter Pyruvat-Carboxylase-Aktivität kodiert, verwendet wird.

34. Verwendung nach Anspruch 32 oder 33,
**dadurch gekennzeichnet,**
**daß** der die entsprechende Aminosäure produzierende Mikroorganismus mit einem Genkonstrukt, das ein Pyruvat-Carboxylase-Gen enthält, transformiert wird.

35. Verwendung nach Anspruch 34,
**dadurch gekennzeichnet,**
**daß** das Genkonstrukt zusätzlich regulatorische Gensequenzen enthält.

36. Verwendung nach einem der Ansprüche 32 bis 35,
**dadurch gekennzeichnet,**
**daß** ein Pyruvat-Carboxylase-Gen aus Corynebacterium verwendet wird.

37. Verwendung nach einem der Ansprüche 32 bis 36,
**dadurch gekennzeichnet,**
**daß** als Aminosäure-produzierender Mikroorganismus Corynebacterium verwendet wird.

## Claims

1. A process for the microbial production of amino acids of the aspartate and/or glutamate family, in which pyruvate carboxylase activity is increased by genetic modification of the enzyme and/or pyruvate carboxylase gene expression of a microorganism producing the corresponding amino acid.

2. A process according to claim 1,
**characterised in that**
an enzyme with greater pyruvate carboxylase activity is produced by mutation of the endogenous pyruvate carboxylase gene.

3. A process according to claim 1 or claim 2,
**characterised in that**
gene expression of the pyruvate carboxylase is increased by increasing the gene copy number.

4. A process according to claim 3,
**characterised in that**
the pyruvate carboxylase gene is incorporated into a gene construct in order to increase the gene copy number.

5. A process according to claim 4,
**characterised in that**
the gene is incorporated into a gene construct which contains regulatory gene sequences assigned to the pyruvate carboxylase gene.

6. A process according to claim 4 or claim 5,
**characterised in that**
a microorganism producing the corresponding amino acid is transformed with the gene construct containing the gene.

7. A process according to claim 6,
**characterised in that**
a microorganism of the genus Corynebacterium is transformed with the gene construct containing the gene.

8. A process according to claim 6 or claim 7,
**characterised in that**
a microorganism is used for the transformation in which the enzymes involved in the synthesis of the corresponding amino acid are deregulated and/or exhibit elevated export carrier activity for the corresponding amino acid.

9. A process according to any one of claims 6 to 8,
**characterised in that**
a microorganism is used for the transformation which contains an elevated proportion of the central metabolism metabolites involved in the synthesis of the corresponding amino acid.

10. A process according to any one of claims 6 to 9,
**characterised in that**
a microorganism is used for the transformation in which a biosynthetic pathway competitive with the biosynthetic pathway for the corresponding amino acid proceeds with reduced activity.

11. A process according to any one of the preceding claims,
**characterised in that**
the pyruvate carboxylase gene is isolated from a microorganism strain of the genus Corynebacterium.

12. A process according to any one of the preceding claims,
**characterised in that**
gene expression is increased by enhancement of the transcription signals.

13. A process according to any one of the preceding claims,
**characterised in that**
the tac promoter is located upstream from the pyruvate carboxylase gene.

14. A process according to claim 13,
**characterised by**
regulatory sequences assigned to the tac promoter.

15. A process according to any one of the preceding claims,
**characterised in that**
the pyruvate carboxylase gene used is a gene with the amino acid sequence stated under SEQ ID no. 2 and the nucleotide sequence coding the allele variations thereof.

16. A process according to claim 15,
**characterised in that**
the pyruvate carboxylase gene used is a gene with the nucleotide sequence from nucleotide 165 to 3587 according to SEQ ID no. 1 or a substantially equivalently acting DNA sequence.

17. A process according to any one of the preceding claims for the production of lysine, threonine, homoserine, glutamate and/or arginine.

18. A pyruvate carboxylase gene with an amino acid sequence stated under SEQ ID no. 2 and/or a nucleotide sequence coding the allele variations thereof.

19. A pyruvate carboxylase gene according to claim 18 with the nucleotide sequence from nucleotide 165 to 3587 according to SEQ ID no. 1 or a substantially equivalently acting DNA sequence.

20. A pyruvate carboxylase gene according to claim 18 or claim 19 with an upstream promoter of the nucleotide sequence from nucleotide 20 to 109 according to SEQ ID no. 1 or a substantially equivalently acting DNA sequence.

21. A pyruvate carboxylase gene according to claim 18 or claim 19 with an upstream tac promoter.

22. A pyruvate carboxylase gene according to claim 21 with regulatory sequences assigned to the promoter.

23. A pyruvate carboxylase gene according to any one of claims 18 to 20 with these assigned regulatory gene sequences.

24. A gene structure containing a pyruvate carboxylase gene according to any one of claims 18 to 23.

25. A vector containing a pyruvate carboxylase gene according to any one of claims 18 to 23 or a gene structure according to claim 24.

26. A transformed cell containing in replicable form a pyruvate carboxylase gene according to any one of claims 18 to 23 or a gene structure according to claim 24.

27. A transformed. cell according to claim 26 containing a vector according to claim 25.

28. A transformed cell according to claim 26 or claim 27,
**characterised in that**
it belongs to the genus Corynebacterium.

29. A transformed cell according to any one of claims 26 to 28,
**characterised in that**
the enzymes therein involved in synthesis of the corresponding amino acid and/or the enzymes involved in the export of the corresponding amino acid are deregulated.

30. A transformed cell according to any one of claims 26 to 29,
**characterised in that**
it contains an elevated proportion of central metabolism metabolites involved in the synthesis of the corresponding amino acid.

31. A transformed cell according to any one of claims 26 to 30,
**characterised in that**
it contains a reduced proportion of central metabolism metabolites not involved in the synthesis of the corresponding amino acid.

32. Use of a pyruvate carboxylase gene to increase the production of amino acids originating from the aspartate and/or glutamate family by microorganisms.

33. Use according to claim 32,
**characterised in that**
a mutated pyruvate carboxylase gene, which codes for an enzyme with elevated pyruvate carboxylase activity, is used.

34. Use according to claim 32 or claim 33,
**characterised in that**
the microorganism producing the corresponding amino acid is transformed with a gene construct which contains a pyruvate carboxylase gene.

35. Use according to claim 34,
**characterised in that**
the gene construct additionally contains regulatory gene sequences.

36. Use according to any one of claims 32 to 35,
**characterised in that**
a pyruvate carboxylase gene from Corynebacterium is used.

37. Use according to any one of claims 32 to 36,
**characterised in that**
Corynebacterium is used as the amino acid producing microorganism.

## Revendications

1. Procédé pour la fabrication microbienne d'acides aminés de la famille des aspartates et/ou des glutamates,
selon lequel
on augmente l'activité pyruvate carboxylase par modification génétique de l'enzyme et/ou l'expression génétique de la pyruvate carboxylase d'un micro-organisme produisant l'acide aminé correspondant.

2. Procédé selon la revendication 1,
**caractérisé en ce qu'**
on produit une enzyme présentant une activité pyruvate carboxylase plus importante par la mutation du gène pyruvate carboxylase endogène.

3. Procédé selon la revendication 1 ou 2,
**caractérisé en ce que**
l'expression génétique de la pyruvate carboxylase est augmentée par l'augmentation du nombre de copies génétiques.

4. Procédé selon la revendication 3,
**caractérisé en ce que**
le gène pyruvate carboxylase est inséré dans une construction génétique pour l'augmentation du nombre de copies génétiques.

5. Procédé selon la revendication 4,
**caractérisé en ce que**
le gène est inséré dans une construction génétique qui contient des séquences génétiques régulatrices associées au gène pyruvate carboxylase.

6. Procédé selon la revendication 4 ou 5,
**caractérisé en ce qu'**
un micro-organisme produisant l'acide aminé correspondant est transformé avec la construction génétique contenant le gène.

7. Procédé selon la revendication 6,
**caractérisé en ce qu'**
un micro-organisme de l'espèce Corynebacterium est transformé avec la construction génétique contenant le gène.

8. Procédé selon la revendication 6 ou 7,
**caractérisé en ce qu'**
on utilise pour la transformation, un micro-organisme dans lequel les enzymes prenant part à la synthèse de l'acide aminé correspondant sont dérégulés et/ou présentent une activité de vecteur d'exportation élevée pour l'acide aminé correspondant.

9. Procédé selon l'une quelconque des revendications 6 à 8,
**caractérisé en ce que**
pour la transformation, on utilise un micro-organisme qui contient une proportion élevée de métabolites du métabolisme central prenant part à la synthèse de l'acide aminé correspondant.

10. Procédé selon l'une quelconque des revendications 6 à 9,
**caractérisé en ce que**
pour la transformation on utilise un micro-organisme dans lequel une voie de biosynthèse concurrentielle à la voie de biosynthèse de l'acide aminé correspondant se termine avec une activité réduite.

11. Procédé selon l'une quelconque des revendications précédentes,
**caractérisé en ce que**
le gène pyruvate carboxylase est isolé à partir d'une source de micro-organisme de l'espèce Corynebacterium.

12. Procédé selon l'une quelconque des revendications précédentes,
**caractérisé en ce que**
l'expression génétique est augmentée par le renforcement des signaux de la transcriptase.

13. Procédé selon l'une quelconque des revendications précédentes,
**caractérisé en ce que**
le promoteur tac est connecté en amont du gène pyruvate carboxylase.

14. Procédé selon la revendication 13,
**caractérisé par**
des séquences régulatrices associées au promoteur tac.

15. Procédé selon l'une quelconque des revendications précédentes,
**caractérisé en ce que**
comme gène pyruvate carboxylase, on utilise un gène présentant une séquence nucléotidique codant pour la séquence d'acides aminés désignée sous SEQ ID NO : 2 et pour ses variations d'allèles.

16. Procédé selon la revendication 15,
**caractérisé en ce que**
comme gène pyruvate carboxylase, on utilise un gène présentant une séquence nucléotidique de nucléotides 165 à 3587 selon la SEQ ID NO : 1 ou une séquence d'ADN présentant un effet essentiellement similaire.

17. Procédé selon l'une quelconque des revendications précédentes pour la fabrication de lysine, thréonine, homosérine, glutamate et/ou arginine.

18. Gène pyruvate carboxylase avec une séquence nucléotidique codant pour la séquence d'acides aminés désignée sous SEQ ID NO : 2 et/ou ses variations d'allèle.

19. Gène pyruvate carboxylase selon la revendication 18, avec la séquence nucléotidique de nucléotides 165 à 3587 selon SEQ ID NO : 1 ou une séquence d'ADN présentant un effet essentiellement similaire.

20. Gène pyruvate carboxylase selon la revendication 18 ou 19, présentant un promoteur connecté en amont de la séquence nucléotidique des nucléotides 20 à 109 selon SEQ ID NO : 1 ou une séquence d'ADN présentant un effet essentiellement similaire.

21. Gène pyruvate carboxylase selon la revendication 18 ou 19, avec un promoteur tac connecté en amont.

22. Gène pyruvate carboxylase selon la revendication 21, ayant des séquences régulatrices associées au promoteur.

23. Gène pyruvate carboxylase selon l'une quelconque des revendications 18 à 20, avec des séquences génétiques régulatrices associées à celui-ci.

24. Structure génétique contenant un gène pyruvate carboxylase selon l'une quelconque des revendications 18 à 23.

25. Vecteur contenant un gène pyruvate carboxylase selon l'une quelconque des revendications 18 à 23, ou une structure génétique selon la revendication 24.

26. Cellule transformée contenant, dans une forme pouvant être répliquée, un gène pyruvate carboxylase selon l'une quelconque des revendications 18 à 23, ou une structure génétique selon la revendication 24.

27. Cellule transformée selon la revendication 26, contenant un vecteur selon la revendication 25.

28. Cellule transformée selon la revendication 26 ou 27,
**caractérisée en ce qu'**
elle appartient à l'espèce Corynebacterium.

29. Cellule transformée selon l'une quelconque des revendications 26 à 28,
**caractérisée en ce que**
dans celle-ci, les enzymes prenant part à la synthèse des acides aminés correspondants et/ou les enzymes prenant part à l'export des acides aminés correspondants sont dérégulées.

30. Cellule transformée selon l'une quelconque des revendications 26 à 29,
**caractérisée en ce qu'**
elle contient une proportion élevée de métabolites du métabolisme central prenant part à la synthèse de l'acide aminé correspondant.

31. Cellule transformée selon l'une quelconque des revendications 26 à 30,
**caractérisée en ce qu'**
elle contient une proportion réduite en métabolites du métabolisme central ne prenant pas part à la synthèse de l'acide aminé correspondant.

32. Utilisation d'un gène pyruvate carboxylase, pour l'augmentation de la production des acides aminés de micro-organismes provenant de la famille des aspartates et/ou des glutamates.

33. Utilisation selon la revendication 32,
**caractérisée en ce qu'**
on utilise un gène pyruvate carboxylase muté qui code pour un enzyme présentant une activité pyruvate carboxylase plus importante.

34. Utilisation selon la revendication 32 ou 33,
**caractérisée en ce que**
le micro-organisme produisant l'acide aminé correspondant est transformé avec une construction génétique qui contient un gène pyruvate carboxylase.

35. Utilisation selon la revendication 34,
**caractérisée en ce que**
la construction génétique contient en plus des séquences génétiques régulatrices.

36. Utilisation selon l'une quelconque des revendications 32 à 35,
**caractérisée en ce qu'**
on utilise un gène pyruvate carboxylase de l'espèce Corynebacterium.

37. Utilisation selon l'une quelconque des revendications 32 à 36,
**caractérisée en ce que**
comme micro-organisme produisant l'acide aminé, on utilise le Corynebacterium.


### SEQUENZPROTOKOLL

(1) ALLGEMEINE ANGABEN:
   (i) ANMELDER:
      (A) NAME: Forschungszentrum Juelich GmbH
      (B) STRASSE: Postfach 1913
      (C) ORT: Juelich
      (E) LAND: Deutschland
      (F) POSTLEITZAHL: 52425
   (ii) BEZEICHNUNG DER ERFINDUNG: Pyruvat Carboxylase
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 3728 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 1:
   CGCAACCGTG CTTGAAGTCG TGCAGGTCAG GGGAGTGTTG CCCGAAAACA TTGAGAGGAA      60
   AACAAAAACC GATGTTTGAT TGGGGGAATC GGGGGTTACG ATACTAGGAC GCAGTGACTG     120
   CTATCACCCT TGGCGGTCTC TTGTTGAAAG GAATAATTAC TCTAGTGTCG ACTCACACAT     180
   CTTCAACGCT TCCAGCATTC AAAAAGATCT TGGTAGCAAA CCGCGGCGAA ATCGCGGTCC     240
   GTGCTTTCCG TGCAGCACTC GAAACCGGTG CAGCCACGGT AGCTATTTAC CCCCGTGAAG     300
   ATCGGGGATC ATTCCACCGC TCTTTTGCTT CTGAAGCTGT CCGCATTGGT ACCGAAGGCT     360
   CACCAGTCAA GGCGTACCTG GACATCGATG AAATTATCGG TGCAGCTAAA AAAGTTAAAG     420
   CAGATGCCAT TTACCCGGGA TACGGCTTCC TGTCTGAAAA TGCCCAGCTT GCCCGCGAGT     480
   GTGCGGAAAA CGGCATTACT TTTATTGGCC CAACCCCAGA GGTTCTTGAT CTCACCGGTG 540
   ATAAGTCTCG CGCGGTAACC GCCGCGAAGA AGGCTGGTCT GCCAGTTTTG GCGGAATCCA 600
   CCCCGAGCAA AAACATCGAT GAGATCGTTA AAAGCGCTGA AGGCCAGACT TACCCCATCT 660
   TTGTGAAGGC AGTTGCCGGT GGTGGCGGAC GCGGTATGCG TTTTGTTGCT TCACCTGATG 720
   AGCTTCGCAA ATTAGCAACA GAAGCATCTC GTGAAGCTGA AGCGGCTTTC GGCGATGGCG 780
   CGGTATATGT CGAACGTGCT GTGATTAACC CTCAGCATAT TGAAGTGCAG ATCCTTGGCG 840
   ATCACACTGG AGAAGTTGTA CACCTTTATG AACGTGACTG CTCACTGCAG CGTCGTCACC 900
   AAAAAGTTGT CGAAATTGCG CCAGCACAGC ATTTGGATCC AGAACTGCGT GATCGCATTT 960
   GTGCGGATGC AGTAAAGTTC TGCCGCTCCA TTGGTTACCA GGGCGCGGGA ACCGTGGAAT 1020
   TCTTGGTCGA TGAAAAGGGC AACCACGTCT TCATCGAAAT GAACCCACGT ATCCAGGTTG 1080
   AGCACACCGT GACTGAAGAA GTCACCGAGG TGGACCTGGT GAAGGCGCAG ATGCGCTTGG 1140
   CTGCTGGTGC AACCTTGAAG GAATTGGGTC TGACCCAAGA TAAGATCAAG ACCCACGGTG 1200
   CAGCACTGCA GTGCCGCATC ACCACGGAAG ATCCAAACAA CGGCTTCCGC CCAGATACCG 1260
   GAACTATCAC CGCGTACCGC TCACCAGGCG GAGCTGGCGT TCGTCTTGAC GGTGCAGCTC 1320
   AGCTCGGTGG CGAAATCACC GCACACTTTG ACTCCATGCT GGTGAAAATG ACCTGCCGTG 1380
   GTTCCGACTT TGAAACTGCT GTTGCTCGTG CACAGCGCGC GTTGGCTGAG TTCACCGTGT 1440
   CTGGTGTTGC AACCAACATT GGTTTCTTGC GTGCGTTGCT GCGGGAAGAG GACTTCACTT 1500
   CCAAGCGCAT CGCCACCGGA TTCATTGCCG ATCACCCGCA CCTCCTTCAG GCTCCACCTG 1560
   CTGATGATGA GCAGGGACGC ATCCTGGATT ACTTGGCAGA TGTCACCGTG AACAAGCCTC 1620
   ATGGTGTGCG TCCAAAGGAT GTTGCAGCTC CTATCGATAA GCTGCCTAAC ATCAAGGATC 1680
   TGCCACTGCC ACGCGGTTCC CGTGACCGCC TGAAGCAGCT TGGCCCAGCC GCGTTTGCTC 1740
   GTGATCTCCG TGAGCAGGAC GCACTGGCAG TTACTGATAC CACCTTCCGC GATGCACACC 1800
   AGTCTTTGCT TGCGACCCGA GTCCGCTCAT TCGCACTGAA GCCTGCGGCA GAGGCCGTCG 1860
   CAAAGCTGAC TCCTGAGCTT TTGTCCGTGG AGGCCTGGGG CGGCGCGACC TACGATGTGG 1920
   CGATGCGTTT CCTCTTTGAG GATCCGTGGG ACAGGCTCGA CGAGCTGCGC GAGGCGATGC 1980
   CGAATGTAAA CATTCAGATG CTGCTTCGCG GCCGCAACAC CGTGGGATAC ACCCCGTACC 2040
   CAGACTCCGT CTGCCGCGCG TTTGTTAAGG AAGCTGCCAG CTCCGGCGTG GACATCTTCC 2100
   GCATCTTCGA CGCGCTTAAC GACGTCTCCC AGATGCGTCC AGCAATCGAC GCAGTCCTGG 2160
   AGACCAACAC CGCGGTAGCC GAGGTGGCTA TGGCTTATTC TGGTGATCTC TCTGATCCAA 2220
   ATGAAAAGCT CTACACCCTG GATTACTACC TAAAGATGGC AGAGGAGATC GTCAAGTCTG 2280
   GCGCTCACAT CTTGGCCATT AAGGATATGG CTGGTCTGCT TCGCCCAGCT GCGGTAACCA 2340
   AGCTGGTCAC CGCACTGCGC CGTGAATTCG ATCTGCCAGT GCACGTGCAC ACCCACGACA 2400
   CTGCGGGTGG CCAGCTGGCA ACCTACTTTG CTGCAGCTCA AGCTGGTGCA GATGCTGTTG 2460
   ACGGTGCTTC CGCACCACTG TCTGGCACCA CCTCCCAGCC ATCCCTGTCT GCCATTGTTG 2520
   CTGCATTCGC GCACACCCGT CGCGATACCG GTTTGAGCCT CGAGGCTGTT TCTGACCTCG 2580
   AGCCGTACTG GGAAGCAGTG CGCGGACTGT ACCTGCCATT TGAGTCTGGA ACCCCAGGCC 2640
   CAACCGGTCG CGTCTACCGC CACGAAATCC CAGGCGGACA GTTGTCCAAC CTGCGTGCAC 2700
   AGGCCACCGC ACTGGGCCTT GCGGATCGTT TCGAACTCAT CGAAGACAAC TACGCAGCCG 2760
   TTAATGAGAT GCTGGGACGC CCAACCAAGG TCACCCCATC CTCCAAGGTT GTTGGCGACC 2820
   TCGCACTCCA CCTCGTTGGT GCGGGTGTGG ATCCAGCAGA CTTTGCTGCC GATCCACAAA 2880
   AGTACGACAT CCCAGACTCT GTCATCGCGT TCCTGCGCGG CGAGCTTGGT AACCCTCCAG 2940
   GTGGCTGGCC AGAGCCACTG CGCACCCGCG CACTGGAAGG CCGCTCCGAA GGCAAGGCAC 3000
   CTCTGACGGA AGTTCCTGAG GAAGAGCAGG CGCACCTCGA CGCTGATGAT TCCAAGGAAC 3060
   GTCGCAATAG CCTCAACCGC CTGCTGTTCC CGAAGCCAAC CGAAGAGTTC CTCGAGCACC 3120
   GTCGCCGCTT CGGCAACACC TCTGCGCTGG ATGATCGTGA ATTCTTCTAC GGCCTGGTCG 3180
   AAGGCCGCGA GACTTTGATC CGCCTGCCAG ATGTGCGCAC CCCACTGCTT GTTCGCCTGG 3240
   ATGCGATCTC TGAGCCAGAC GATAAGGGTA TGCGCAATGT TGTGGCCAAC GTCAACGGCC 3300
   AGATCCGCCC AATGCGTGTG CGTGACCGCT CCGTTGAGTC TGTCACCGCA ACCGCAGAAA 3360
   AGGCAGATTC CTCCAACAAG GGCCATGTTG CTGCACCATT CGCTGGTGTT GTCACCGTGA 3420
   CTGTTGCTGA AGGTGATGAG GTCAAGGCTG GAGATGCAGT CGCAATCATC GAGGCTATGA 3480
   AGATGGAAGC AACAATCACT GCTTCTGTTG ACGGCAAAAT CGATCGCGTT GTGGTTCCTG 3540
   CTGCAACGAA GGTGGAAGGT GGCGACTTGA TCGTCGTCGT TTCCTAAACC TTTCTGTAAA 3600
   AAGCCCCGCG TCTTCCTCAT GGAGGAGGCG GGGCTTTTTG GGCCAAGATG GGAGATGGGT 3660
   GAGTTGGATT TGGTCTGATT CGACACTTTT AAGGGCAGAG ATTTGAAGAT GGAGACCAAG 3720
   GCTCAAAG 3728
(2) ANGABEN ZU SEQ ID NO: 2:
   (i) SEQUENZKENNZEICHEN:
      (A) LÄNGE: 1140 Aminosäuren
      (B) ART: Aminosäure
      (C) STRANGFORM: Einzelsträng
      (D) TOPOLOGIE: linear
   (ii) ART DES MOLEKÜLS: Protein
   (xi) SEQUENZBESCHREIBUNG: SEQ ID NO: 2:
   Met Ser Thr His Thr Ser Ser Thr Leu Pro Ala Phe Lys Lys Ile Leu
   1               5                   10                  15
   Val Ala Asn Arg Gly Glu Ile Ala Val Arg Ala Phe Arg Ala Ala Leu
               20                  25                  30
   Glu Thr Gly Ala Ala Thr Val Ala Ile Tyr Pro Arg Glu Asp Arg Gly
           35                  40                  45
   Ser Phe His Arg Ser Phe Ala Ser Glu Ala Val Arg Ile Gly Thr Glu
       50                  55                  60
   Gly Ser Pro Val Lys Ala Tyr Leu Asp Ile Asp Glu Ile Ile Gly Ala
   65                  70                  75                  80
   Ala Lys Lys Val Lys Ala Asp Ala Ile Tyr Pro Gly Tyr Gly Phe Leu
                   85                  90                  95
   Ser Glu Asn Ala Gln Leu Ala Arg Glu Cys Ala Glu Asn Gly Ile Thr
               100                 105                 110
   Phe Ile Gly Pro Thr Pro Glu Val Leu Asp Leu Thr Gly Asp Lys Ser
           115                 120                 125
   Arg Ala Val Thr Ala Ala Lys Lys Ala Gly Leu Pro Val Leu Ala Glu
       130                 135                 140
   Ser Thr Pro Ser Lys Asn Ile Asp Glu Ile Val Lys Ser Ala Glu Gly
   145                 150                 155                 160
   Gln Thr Tyr Pro Ile Phe Val Lys Ala Val Ala Gly Gly Gly Gly Arg
                   165                 170                 175
   Gly Met Arg Phe Val Ala Ser Pro Asp Glu Leu Arg Lys Leu Ala Thr
               180                 185                 190
   Glu Ala Ser Arg Glu Ala Glu Ala Ala Phe Gly Asp Gly Ala Val Tyr
   195 200 205
   Val Glu Arg Ala Val Ile Asn Pro Gln His Ile Glu Val Gln Ile Leu
   210 215 220
   Gly Asp His Thr Gly Glu Val Val His Leu Tyr Glu Arg Asp Cys Ser
   225 230 235 240
   Leu Gln Arg Arg His Gln Lys Val Val Glu Ile Ala Pro Ala Gln His
   245 250 255
   Leu Asp Pro Glu Leu Arg Asp Arg Ile Cys Ala Asp Ala Val Lys Phe
   260 265 270
   Cys Arg Ser Ile Gly Tyr Gln Gly Ala Gly Thr Val Glu Phe Leu Val
   275 280 285
   Asp Glu Lys Gly Asn His Val Phe Ile Glu Met Asn Pro Arg Ile Gln
   290 295 300
   Val Glu His Thr Val Thr Glu Glu Val Thr Glu Val Asp Leu Val Lys
   305 310 315 320
   Ala Gln Met Arg Leu Ala Ala Gly Ala Thr Leu Lys Glu Leu Gly Leu
   325 330 335
   Thr Gln Asp Lys Ile Lys Thr His Gly Ala Ala Leu Gln Cys Arg Ile
   340 345 350
   Thr Thr Glu Asp Pro Asn Asn Gly Phe Arg Pro Asp Thr Gly Thr Ile
   355 360 365
   Thr Ala Tyr Arg Ser Pro Gly Gly Ala Gly Val Arg Leu Asp Gly Ala
   370 375 380
   Ala Gln Leu Gly Gly Glu Ile Thr Ala His Phe Asp Ser Met Leu Val
   385 390 395 400
   Lys Met Thr Cys Arg Gly Ser Asp Phe Glu Thr Ala Val Ala Arg Ala
   405 410 415
   Gln Arg Ala Leu Ala Glu Phe Thr Val Ser Gly Val Ala Thr Asn Ile
   420 425 430
   Gly Phe Leu Arg Ala Leu Leu Arg Glu Glu Asp Phe Thr Ser Lys Arg
   435 440 445
   Ile Ala Thr Gly Phe Ile Ala Asp His Pro His Leu Leu Gln Ala Pro
   450 455 460
   Pro Ala Asp Asp Glu Gln Gly Arg Ile Leu Asp Tyr Leu Ala Asp Val
   465 470 475 480
   Thr Val Asn Lys Pro His Gly Val Arg Pro Lys Asp Val Ala Ala Pro
   485 490 495
   Ile Asp Lys Leu Pro Asn Ile Lys Asp Leu Pro Leu Pro Arg Gly Ser
   500 505 510
   Arg Asp Arg Leu Lys Gln Leu Gly Pro Ala Ala Phe Ala Arg Asp Leu
   515 520 525
   Arg Glu Gln Asp Ala Leu Ala Val Thr Asp Thr Thr Phe Arg Asp Ala
   530 535 540
   His Gln Ser Leu Leu Ala Thr Arg Val Arg Ser Phe Ala Leu Lys Pro
   545 550 555 560
   Ala Ala Glu Ala Val Ala Lys Lys Thr Pro Glu Leu Leu Ser Val Glu
   565 570 575
   Ala Trp Gly Gly Ala Thr Tyr Asp Val Ala Met Arg Phe Leu Phe Glu
   580 585 590
   Asp Pro Trp Asp Arg Leu Asp Glu Leu Arg Glu Ala Met Pro Asn Val
   595 600 605
   Asn Ile Gln Met Leu Leu Arg Gly Arg Asn Thr Val Gly Tyr Thr Pro
   610 615 620
   Tyr Pro Asp Ser Val Cys Arg Ala Phe Val Lys Glu Ala Ala Ser Ser
   625 630 635 640
   Gly Val Asp Ile Phe Arg Ile Phe Asp Ala Leu Asn Asp Val Ser Gln
   645 650 655
   Met Arg Pro Ala Ile Asp Ala Val Leu Glu Thr Asn Thr Ala Val Ala
   660 665 670
   Glu Val Ala Met Ala Tyr Ser Gly Asp Leu Ser Asp Pro Asn Glu Lys
   675 680 685
   Leu Tyr Thr Leu Asp Tyr Tyr Leu Lys Met Ala Glu Glu Ile Val Lys
   690 695 700
   Ser Gly Ala His Ile Leu Ala Ile Lys Asp Met Ala Gly Leu Leu Arg
   705 710 715 720
   Pro Ala Ala Val Thr Lys Leu Val Thr Ala Leu Arg Arg Glu Phe Asp
   725 730 735
   Leu Pro Val His Val His Thr His Asp Thr Ala Gly Gly Gln Leu Ala
   740 745 750
   Thr Tyr Phe Ala Ala Ala Gln Ala Gly Ala Asp Ala Val Asp Gly Ala
   755 760 765
   Ser Ala Pro Leu Ser Gly Thr Thr Ser Gln Pro Ser Leu Ser Ala Ile
   770 775 780
   Val Ala Ala Phe Ala His Thr Arg Arg Asp Thr Gly Leu Ser Leu Glu
   785 790 795 800
   Ala Val Ser Asp Leu Glu Pro Tyr Trp Glu Ala Val Arg Gly Leu Tyr
   805 810 815
   Leu Pro Phe Glu Ser Gly Thr Pro Gly Pro Thr Gly Arg Val Tyr Arg
   820 825 830
   His Glu Ile Pro Gly Gly Gln Leu Ser Asn Leu Arg Ala Gln Ala Thr
   835 840 845
   Ala Leu Gly Leu Ala Asp Arg Phe Glu Leu Ile Glu Asp Asn Tyr Ala
   850 855 860
   Ala Val Asn Glu Met Leu Gly Arg Pro Thr Lys Val Thr Pro Ser Ser
   865 870 875 880
   Lys Val Val Gly Asp Leu Ala Leu His Leu Val Gly Ala Gly Val Asp
   885 890 895
   Pro Ala Asp Phe Ala Ala Asp Pro Gln Lys Tyr Asp Ile Pro Asp Ser
   900 905 910
   Val Ile Ala Phe Leu Arg Gly Glu Leu Gly Asn Pro Pro Gly Gly Trp
   915 920 925
   Pro Glu Pro Leu Arg Thr Arg Ala Leu Glu Gly Arg Ser Glu Gly Lys
   930 935 940
   Ala Pro Leu Thr Glu Val Pro Glu Glu Glu Gln Ala His Leu Asp Ala
   945 950 955 960
   Asp Asp Ser Lys Glu Arg Arg Asn Ser Leu Asn Arg Leu Leu Phe Pro
   965 970 975
   Lys Pro Thr Glu Glu Phe Leu Glu His Arg Arg Arg Phe Gly Asn Thr
   980 985 990
   Ser Ala Leu Asp Asp Arg Glu Phe Phe Tyr Gly Leu Val Glu Gly Arg
   995 1000 1005
   Glu Thr Leu Ile Arg Leu Pro Asp Val Arg Thr Pro Leu Leu Val Arg
   1010 1015 1020
   Leu Asp Ala Ile Ser Glu Pro Asp Asp Lys Gly Met Arg Asn Val Val
   1025 1030 1035 1040
   Ala Asn Val Asn Gly Gln Ile Arg Pro Met Arg Val Arg Asp Arg Ser
   1045 1050 1055
   Val Glu Ser Val Thr Ala Thr Ala Glu Lys Ala Asp Ser Ser Asn Lys
                       1060                1065                1070
   Gly His Val Ala Ala Pro Phe Ala Gly Val Val Thr Val Thr Val Ala
               1075                1080                1085
   Glu Gly Asp Glu Val Lys Ala Gly Asp Ala Val Ala Ile Ile Glu Ala
           1090                1095                1100
   Met Lys Met Glu Ala Thr Ile Thr Ala Ser Val Asp Gly Lys Ile Asp
   1105                1110                1115                1120
   Arg Val Val Val Pro Ala Ala Thr Lys Val Glu Gly Gly Asp Leu Ile
                   1125                1130                1135
   Val Val Val Ser
               1140